# EUROPEAN PATENT SPECIFICATION

(11) **EP 0 461 827 B1**
(45) Date of publication and mention of the grant of the patent: **17.09.1997**
(21) Application number: 91305186.8
(22) Date of filing: 10.06.1991
(51) Int. Cl.: A61K 7/06

(54) **Hair restorer**
Haarregenerierendes Mittel
Agent de régéneration des cheveux

(30) Priority: 11.06.1990 JP 152319/90
(43) Date of publication of application: 18.12.1991
(73) Proprietor: KABUSHIKI KAISHA HAYASHIBARA SEIBUTSU KAGAKU KENKYUJO, Okayama-shi Okayama (JP)
(72) Inventor: Miyake, Toshio, Okayama-shi, Okayama (JP)
(74) Representative: Pendlebury, Anthony

(56) References cited:
- EP-A- 0 277 428
- EP-A- 0 387 042
- EP-A- 0 398 484
- EP-A- 0 402 049
- DE-A- 1 910 561
- DATABASE WPI Week 8921, Derwent Publications Ltd., London, GB; AN 89-155044
- DATABASE WPI Week 8728, Derwent Publications Ltd., London, GB; AN 87-197194
- DATABASE WPI Week 8201, Derwent Publications Ltd., London, GB; AN 82-01054E
- THE JOURNAL OF BIOCHEMISTRY vol. 107, no. 2, January 1990, JAPAN pages 222 - 227 NORIO MUTO ET AL 'Enzymatic Formation of a Nonreducing L-Ascorbic Acid alpha-Glycoside: Purification and Properties of alpha-Glycosidases Catalysing Site-Specific Transglucosylation from Rat Small Intestine'

## Description

The present invention relates to a hair restorer.

Although various factors have beer speculated for the occurrence of alopecias, to sum up, the direct certain factor hinders hair to achieve its normal cycle, and allows the folliculus pili to stay in the telogen stage. Therefore, the folliculus pili in the telogen stage should be changed into the normal anagen stage in order to improve alopecias and promote the growth and regeneration of hair.

To improve alopecias and promote the growth and regeneration of hair, various types of hair restorers have been proposed, which are roughly classified into the following types based on their functions:
(i) A type which promotes the growth and regeneration of hair by removing unnecessary keratin and epidermis around alopecic sites, and inhibiting the inflammation around the alopecic sites;
(ii) Another type which promotes the growth and regeneration of hair by activating hair-matrix cells at their cell level; and
(iii)Still another type which promotes the growth and regeneration of hair by accelerating the metabolism of the scalp or supplementing nutritions to the scalp.

Since, usually, such hair restorers are continually used over a long period of time and not restricted on their administration frequency and dose specifically, it is an important factor that the hair restorers should be free from unsatisfiable side effects, to say nothing of an exertion of satisfiable efficacy. Hair restorers of type (i) have the drawback that an excessive amount of administration may induce a skin inflammatory and more augment the symptom of alopecias because the hair restorers contain a bactericide, anti-inflammatory, and dissolvent of keratin such as salicylic acid, resorcinol and glycyrrhizin. Hair restorers of type (ii) also have the drawback similar to that of the hair restorers of type (i) because the hair restorers of type (ii) contain ingredients such as mononitroguaiacol, derivatives of pantothenic acid, female sex hormones and amino acids, all of which have a relatively strong pharmacological-activity.

Since the hair restorers of type (iii) contain nutritional agents and vasodilators or blood-circulation promoting agents such as carpronium chloride, Japanese chiretta extract (swertiamarin), iodized garlic extract, vitamin E and L-ascorbic acid, the hair restorers of type (iii) have the advantages that they act moderately with satisfiable handleability and less fear of unsatisfiable side effects, but the hair restorers have the drawback that the treatment of alopecias with the hair restorers requires a relatively long period of time, or the expected result may- not be attained because their activity is relatively low.

Hair restores of type (iii) have a great feature that they exhibit a relatively moderate effect and cause no unsatisfiable side effect because they contain a physiologically active substance such as L-ascorbic acid or vitamin E which are essential nutrients. For example, Japanese Patent Laid-Open No.142,108/87 discloses the preparation of the hair restorer and the treatment of alopecia therewith, wherein said hair restorer contains vitamin E and L-ascorbic acid together with fatty acid- and inorganic ester-derivatives such as L-ascorbic acid monostearate, L-ascorbic acid isopalmitate, L-ascorbic acid dipalmitate, L-ascorbic acid sulfuric ester, and L-ascorbic acid phosphoric ester.

It is well known that L-ascorbic acid has the following drawbacks: (i) It is highly unstable because of its direct reducing activity; (ii) It is readily decomposed through oxidation to lose its physiological activity; and (iii) A relatively long term storage thereof is very difficult, and it readily changes to yellow or reddish brown when- processed into a product. Japanese Patent Laid-Open No.142,108/87 discloses that the hair restorer which contains L-ascorbic acid, vitamin E and their derivatives should be used at a temperature which does not affect their stability.

Furthermore, L-ascorbic acid and vitamin E have the further drawbacks:
(i) The expected effect of the growth and regeneration of hair may not be attained when L-ascorbic acid and vitamin E are used in a hair restorer for external application because their permeability into a deeper part of skin tissue is relatively low; and
(ii) Although the stability of a derivative of L-ascorbic acid in a hair restorer is satisfiable, the derivative could not function as L-ascorbic acid in the body and is excreted from the body as an extraneous substance.

It would be one of our common wishes to keep our head with capilli during our life. Recently, the development of a hair restorer, which is relatively high in safeness and efficacy, is strongly expected because such wish is more and more augmented.

The present invention aims to provide a hair restorer which comprises as the effective ingredient a physiologically active substance having a relatively high-safeness and high-stability, as well as exhibiting a satisfiable effect of the growth and regeneration of hair when applied to human and animals.

The present inventor studied vitamins and their derivatives in order to accomplish this object.

As a result, the present inventor found that vitamins and their derivatives, especially, L-ascorbic acid (vitamin C) and bioflavonoid (vitamin P) exhibited a satisfiable effect of the growth and regeneration of hair when applied to human and animals.

Furthermore, the present inventor found that the permeability of these vitamins and their derivatives into a deeper part of skin tissue was relatively low, and, more particularly the stability of L-ascorbic acid was very low. The L-ascorbic acid, which had permeated into a subcutaneous tissue, could not exhibit the physiological activity inherent to intact L-ascorbic acid because the permeated L-ascorbic acid had been oxidized. Thus, the present inventor concluded that the development of L-ascorbic acid derivatives, which have a relatively high-safeness, improved stability and permeability into a deeper part of skin tissue, was inevitable.

The present inventor studied L-ascorbic acid and bioflavonoid derivatives, in particular, their saccharide derivatives, and found that α-glycosyl-L-ascorbic acids such as α-glucosyl-L-ascorbic acid and α-maltosyl-L-ascorbic acid, and α-glycosyl bioflavonoid such as α-glycosyl rutin, α-glycosyl hesperidin and α-glycosyl naringin were relatively stable, free from unsatisfiable side effects, readily hydrolyzable into L-ascorbic acid and glucose via enzymes in vivo, and satisfiably high in permeability into a deeper part of skin tissue.

The bioflavonoid derivatives such as α-glycosyl hesperidin and α-glycosyl naringin are novel substances which have not been reported in any literature.

Although α-glycosyl-L-ascorbic acid per se is known from Itaru Yamamoto et al, The Journal of Biochemistry, Vol.107, No.2, pp.222-227 (1990), it teaches nothing about the effect of the growth and regeneration of hair.

Although Japanese Patent Publications Nos.32,073/79 and 54,799/83 teach that α-glycosyl rutin exhibits a physiological activity similar to intact rutin in vivo, they do not teach anything about the effect of the growth and regeneration of hair.

By way of example, the present invention will be described in detail hereinafter. The α-glycosyl-L-ascorbic acid usable in the invention has a structure wherein one or more α-D-glucosyl residues are bound in α-1,4 fashion to the hydroxy group of alcohol at the C-2 position in L-ascorbic acid. Particular substances are, for example, 2-O-α-D-glucosyl-L-ascorbic acid, 2-O-α-maltosyl-L-ascorbic acid, 2-O-α-maltotriosyl-L-ascorbic acid, 2-O-α-maltotetraosyl-L-ascorbic acid, 2-O-α-maltopentaosyl-L-ascorbic acid, 2-O-α-maltohexaosyl-L-ascorbic acid, and 2-O-α-maltoheptaosyl-L-ascorbic acid, all of these exhibit no direct reducing activity. The wording "α-glycosyl-L-ascorbic acid" as referred to in the invention is not restricted to those in free acid form, but includes those in salt forms with sodium ion, calcium ion or ferric ion, as long as they cause no special inconvenience. Furthermore, the wording "exhibiting no direct reducing activity" as referred to in the invention means that, unlike the reducing activity of L-ascorbic acid, an activity which does not reduce and decolor 2,6-dichlorophenolindophenol directly.

The wording "α-glycosyl bioflavonoid" as referred to in the invention should include α-glycosyl rutin, α-glycosyl hesperidin and α-glycosyl naringin wherein equimolar or more D-glucose residues are bound in α-fashion to bioflavonoid such as rutin, hesperidin and naringin.

The α-glycosyl bioflavonoid such as α-glycosyl rutin has a structure wherein one or more α-D-glucosyl residues are bound in α-1,4 fashion to rutin (molecular formula: 3-[[6-O-(6-deoxy-α-L-mannopyranosyl)-β-D-glucopyranosyl)]oxy-2-(3,4-dihydroxyphenyl)-5,7-dihydroxy-4H-1-benzopyran-4-one). Particular substances are, for example, α-glucosyl rutin, α-maltosyl rutin, α-maltotriosyl rutin, α-maltotetraosyl rutin and α-maltopentaosyl rutin.

The α-glycosyl hesperidin has a structure wherein one or more α-D-glucosyl residues are bound in α-1,4 fashion to hesperidin (molecular formula: 7-[[6-O-(6-deoxy-α-L-mannopyranosyl)-β-D-glucopyranosyl)]oxy]-2,3-dihydro-5-hydroxy-2-(3-hydroxy-4-methoxypheny)-4H-1-benzopyran-4-one). Particular substances are α-glucosyl hesperidin, α-maltosyl hesperidin, α-maltotriosyl hesperidin, α-maltotetraosyl hesperidin and α-maltopentaosyl hesperidin.

The α-glycosyl naringin has a structure wherein one or more α-D-glucosyl residues are bound in α-1,4 fashion to naringin (molecular formula: 7-[[2-O-(6-deoxy-α-L-mannopyranosyl)-β-D-glucopyranosyl)]oxy]-2,3-dihydro-5-hydroxy-2-(4-hydroxyphenyl)-4H-1-bezopyran-4-one). Particular substances are, for example, α-glucosyl naringin, α-maltosyl naringin, α-maltotriosyl naringin and α-maltotetraosyl naringin.

The α-glycosyl-L-ascorbic acid and α-glycosyl bioflavonoid can be prepared by biochemical- and organic chemical-methods. In general, biochemical methods wherein a saccharide-transferring enzyme such as cyclomaltodextrin glucanotransferase (EC 2.4.1.19), α-amylase (EC 3.2.1.1) and α-glucosidase (EC 3.2.1.20) is allowed to act on a solution containing L-ascorbic acid or bioflavonoid together with α-glucosyl saccharide such as maltooligosaccharide, partial starch hydrolysate, liquefied starch, gelatinized starch and soluble starch are preferred with respect to safeness and economical cost. The α-glycosyl-L-ascorbic acid and α-glycosyl bioflavonoid as the effective ingredient which can be prepared by the methods in Japanese Patent Applications No.274,518/89, entitled "α-Glycosyl-L-ascorbic acid, and its preparation and uses"; No.274,019/89, entitled "Crystalline 2-O-α-D-glucopyranosyl-L-ascorbic acid, and its preparation and uses"; No.217,893/89 (Japanese Patent Laid-Open No.27,293/91), entitled "Process for preparing α-glycosyl rutin and its uses"; No.142,205/89 (Japanese Patent Laid-Open No.58,790/91), entitled "Process for preparing α-glycosyl rutin and its uses"; No.253,269/89, entitled "4^{G}-α-D-Glucopyranosyl hesperidin, and its preparation and uses"; No.141,902/89 (Japanese Patent Laid-Open No.7,593/91), "α-Glycosyl hesperidin, and its preparation and uses"; and No.112,665/90, entitled "α-Glycosyl naringin, and its preparation and uses"; all of which have been made by the present inventor and his coinventors, and the α-glycosyl-L-ascorbic acids and α-glycosyl bioflavonoids can be favorably used in the invention.

The α-glycosyl-L-ascorbic acid or α-glycosyl bioflavonoid prepared by biochemical methods is usually either a mixture of D-glucose, α-glucosyl saccharide, intact L-ascorbic acid, and α-glycosyl derivative wherein one or more α-D-glucosyl residues are bound in α-1,4 fashion to L-ascorbic acid; or a mixture of D-glucose, α-glucosyl saccharide, intact bioflavonoid, α-glycosyl derivative wherein one or more α-D-glucosyl residues are bound in α-1,4 fashion to bioflavonoid. Any mixture can be used in the present hair restorer as long as it contains α-glycosyl-L-ascorbic acid or α-glycosyl bioflavonoid. In a pharmaceutical or cosmetic which requires highly purified- and homogeneous effective-ingredients, concomitants such as intact L-ascorbic acid, bioflavonoid, D-glucose and α-glucosyl saccharide can be separated prior to its use by separating methods which utilize the differences on the molecular weights and affinities of substances. Particular separating methods are membrane separation, column chromatography, high-performance liquid chromatography (HPLC), gel chromatography and ion-exchange chromatography.

Although the content of α-glycosyl-L-ascorbic acid and/or α-glycosyl bioflavonoid in the present hair restorer varies dependently on the type of α-glycosyl-L-ascorbic acid and α-glycosyl bioflavonoid, the type and symptom of alopecias to be treated, and individual difference of recipients, i.e. human and animals, the content of α-glycosyl-L-ascorbic acid and α-glycosyl bioflavonoid is usually in the range of 0.001-10% by weight, preferably, in the range of 0.01-5% by weight.

Although the dose of the hair restorers varies dependently on the content of α-glycosyl-L-ascorbic acid and α-glycosyl bioflavonoid as the effective ingredient, the type and symptom of alopecias to be treated, and individual difference of recipients, i.e. human and animals, the dose is usually in the range of 0.0001-10g/day/adult.

When α-glycosyl-L-ascorbic acid is incorporated as the effective ingredient in the present hair restorer together with α-glycosyl bioflavonoid, the α-glycosyl-L-ascorbic acid and α-glycosyl bioflavonoid synergistically act in vivo to exhibit a higher effect of the growth and regeneration of hair than that of their sole use. Furthermore, a hair restorer containing either α-glycosyl-L-ascorbic acid or α-glycosyl bioflavonoid together with intact L-ascorbic acid and/or bioflavonoid exhibits a satisfiable effect of the growth and regeneration of hair, while the effect of the hair restorer is lower than that of the hair restorer containing α-glycosyl-L-ascorbic acid and α-glycosyl bioflavonoid.

The hair restorer according to the present invention is prepared into an appropriate form, for example, liquid, gelly, emulsion, aerosol or ointment, and is used as a hair tonic, hair liquid, hair lotion, hair cream, hair oil, hair treatment, shampoo and rinse.

In addition to the above essential ingredients, conventional ingredients, for example, an oil- or water-base, emollient, emulsifier, gelatinizer, flavoring agent, antiseptic, antioxidant, coloring agent, refrigerant, bactericide and humectant, which have been used in conventional hair restorers, can be suitably incorporated in the present hair restorer, if necessary. In addition, the present hair restorer can be used in combination with other ingredients, for example, vitamins including vitamin E, hormones, cyanine dyes, amino acids, vasodilators, blood-circulation promoting agents, cell activators, antiinflammatories, bactericides, hyperergic agents for skin, and keratolytics, which have an effect of the growth and regeneration of hair for human and animals.

In particular, the combination of the effective ingredients in the present invention and humectants such as propyleneglycol, 1,3-butyleneglycol, glycerine, sorbitol, 2-octyldodecyl myristate, polyoxyethylene polyoxypropylene glycol, and lauric acid dietharolamine can exhibit a satisfiable effect of the growth and regeneration of hair because the humectants accelerate the dissolution in a solvent, increase the affinity to the skin, and promote the subcutaneous absorbency of the effective ingredients without stimulating the skin.

The cyanine dyes favorably usable in the invention are, for example, "TAKANAL" (6-[2-[(5-bromo-2-pyridyl)amino]vinyl]-1-ethyl-2-picolinium iodide) and "LUMINEX" (2-(2-anilinovinyl)-3,4-dimethyloxazolium iodide), both are commercialized by Nippon Kankoh-Shikiso Kenkyusho Co., Ltd., Okayama, Japan. The combination of the cyanine dyes and the effective ingredients of the present invention can exhibit a synergism, this leads to a relatively high growth and regeneration of hair. The amount of such cyanine dyes to be incorporated is usually in the range of about 0.001-0.01% by weight.

The hair restorer of the invention is commercially valuable because it satisfiably stables over a relatively long period of time, and superior in the promotion of the growth and regeneration of hair when applied to human and animal skin. Thus, the present hair restorer improves the symptom of alopecias and accelerates the growth and regeneration of hair of patients with alopecias such as senile alopecia, alopecia prematura, alopecia areata, mechanical alopecia, and symptomatic alopecia, as well as exhibiting a satisfiable preventive and therapeutic effect on falling out of hair, dandruff, poliosis, and itching of the scalp or skin. Furthermore, the present hair restorer improves the quality of hair or plumage of pet animals such as dog, cat, parrakeet and canary, as well as improving the hair gloss of such animals. The hair restorer also imparts a commercial value to fur-animals such as sheep, fox, alpaca, angora cat, angora rabbit, angora goat, mink and cashmere goat whose hair and fur are utilized.

The present hair restorer is usually administered 1-3 times a day to hair sites. When a hair restorer containing α-glycosyl-L-ascorbic acid which dissociates in an aqueous solution is iontophoresed into affected sites with the low-frequency therapeutic apparatuses as disclosed in Japanese Patent Publication No.41,747/84 and Japanese Patent Laid-Open No.56,060/89, the effect of the growth and regeneration of hair can be more augmented.

It would be speculated that the present hair restorer exhibits the above effects because of the following reasons:
(i) The α-glycosyl-L-ascorbic acid and α-glycosyl bioflavonoid in the present hair restorer have a relatively high-stability and high-hydrophilicity;
(ii) The α-glycosyl-L-ascorbic acid and α-glycosyl bioflavonoid exhibits a relatively high-permeability because they exert a relatively high-affinity to human and animal skin; and
(iii)The α-glycosyl-L-ascorbic acid and α-glycosyl bio-flavonoid are readily hydrolyzable in vivo to exhibit satisfiable physiological activities such as vasodilative activity, blood-circulation promoting activity, and nutrition-supplementing activity.

Conventional hair restorers containing effective ingredients which exhibit a satisfiable effect of the growth and regeneration of hair have the drawback that their actual form is more restricted because their effective ingredients are oil-soluble or water-soluble substances which require an oil-or water-base. The present hair restorer is characteristic in that the form is less restricted because the effective ingredients are readily incorporated both in water- and oil-bases in their effective amounts.

The effect of the growth and regeneration of hair exhibited by the present hair restorer will be described in Experiments and Examples of Preparation, and the wordings of "part(s)" and "%" as referred to in the invention mean "part(s) by weight" and "% by weight" respectively.

### [Experiments]

### Experiment 1

Either of an α-glycosyl-L-ascorbic acid or an α-glycosyl bioflavonoid prepared by the method in Example of Preparation 1-8 was dissolved to give a concentration of 1% in a 50% aqueous ethanol solution containing 2% propyleneglycol, and the mixture was adjusted to pH 6.7 by the addition of 6N sodium hydroxide to obtain a solution in hair lotion form. As control, a solution in hair lotion form was prepared similarly as above, except that α-glycosyl-L-ascorbic acid and α-glycosyl bioflavonoid were replaced with either of intact L-ascorbic acid, bioflavonoid and refined water.

The dorsum surgace of rabbits was sectioned into four areas, 5cm² each, i.e. two areas near at head side and two areas near at tail side which were respectively bisymmetrical about the vertebral columns of the rabbits, and the four areas were depilated by applying thereto silver cream (a depilatory agent) and subjecting them to brief standing. The depilated areas were first sufficiently washed with water, then the depilated areas of the right area near at head side, the left area near at head side, and the right area near at tail side were respectively applied once every day by using a paintbrush with 1ml of the test solution, 1ml of the control solution containing refined water, and 1ml of the control solution containing L-ascorbic acid or bioflavonoid. Furthermore, the left area near at tail side was untreated.

The application of each solution was started on the second day after the depilation, and the newly regenerated hairs were depilated to count on the 10th, 20th and 30th days after the application. The length of 10 coarse hairs regenerated in each area was measured with a micromanipulator, and expressed by the mean remainder of hair length (mm) between the hair length in the area which had been applied with a sample solution and that in the area which had been untreated.

In addition, it has been known that the growth rates of hairs in the bisymmetrical dorsum surfaces about the vertebral column of a rabbit is in the same level, and those in both sexes are also in the same level.

The results were as shown in Table 1.

**Table 1**

| Effective ingredients | Mean remainder of hair length (mm) | | | Judgement |
|---|---|---|---|---|
| | 10th | 20th | 30th (day) | |
| α-Glycosyl-L-ascorbic acid | 0.9 | 2.3 | 3.1 | Present Invention |
| 2-O-α-D-Glucosyl-L-ascorbic acid | 2.4 | 6.8 | 6.9 | Present Invention |
| α-Glycosyl rutin | 1.5 | 3.3 | 4.5 | Present Invention |
| α-Glucosyl rutin | 2.5 | 8.2 | 7.6 | Present Invention |
| α-Glycosyl hesperidin | 1.2 | 1.9 | 4.1 | Present Invention |
| α-Glucosyl hesperidin | 2.3 | 7.4 | 6.7 | Present Invention |
| α-Glycosyl naringin | 0.7 | 2.9 | 3.9 | Present Invention |
| α-Glucosyl naringin | 2.1 | 6.6 | 6.3 | Present Invention |
| L-Ascorbic acid | 0.1 | -0.2 | 0.5 | Control |
| Rutin | 0.3 | 0.9 | 0.5 | Control |
| Hesperidin | 0.1 | 0.5 | 0.2 | Control |
| Naringin | 0.1 | 0.5 | -0.2 | Control |
| Refined water | 0.1 | -0.1 | 0.01 | Control |

As evident from the results in Table 1, a satisfiable effect of the growth and regeneration of hair was found in the groups of the areas which had been applied with either the sample solution containing α-glycosyl-L-ascorbic acid or the sample solution containing α-glycosyl bioflavonoid, but no statistical significance was found between the group of the areas which had been untreated and the group which had been applied with either of intact L-ascorbic acid, bioflavonoid and refined water.

### Experiment 2

Based on the results in Experiment 1, 20 randomly chosen volunteers with alopecias (10 men and 10 women) as the recipient received 3-month clinical test. The volunteers were 15-66 years old.

The symptom of the volunteers, who had received a pharmacotherapy or physiotherapy at a department of dermatology, were the male alopecia such as senile alopecia and alopecia prematura, and alopecia areata such as multiple alopecia areata and malignant alopecia areata.

Since the symptom of the volunteers with a slight alopecia areata may be spontaneously recovered, the volunteers with a relatively small alopecic site and those with the atrophia cutis or atrophic pores which were not clinically observed were excluded in this Experiment.

The procedure of therapeutic treatment used in this Experiment is carried out according to conventional procedure:

Forty-five parts of ethanol, 2 parts of glycerine, 55 parts of refined water, and 1.0 part of 2-O-α-D-glucosyl-L-ascorbic acid or α-glucosyl bioflavonoid prepared by the method in Example of Preparation 2, 4, 6 or 8, were mixed, and the mixture was adjusted to pH 6.7 by the addition of 6N sodium hydroxide to obtain a hair restorer for external application in lotion form. When the volunteers received a physical examination, their affected sites were applied a thin coat of the hair restorer, massaged and exposed to an artificial sunlight. Furthermore, the volunteers were instructed to apply the hair restorer to the affected sites 3 times a day at home.

The efficacy of the growth and regeneration of hair attained by the hair restorer was evaluated based on the four ranks, i.e. "recovery (hair was newly regenerated to exhibit apparently free from alopecia)", "moderate recovery (though the rate of hair regeneration was moderate, no recurrence of alopecia was found)", "not effected (no regeneration of hair was found)", and "unfavorable (side effect or the acceleration of alopecia was found)".

As control, a hair restorer for external application containing intact L-ascorbic acid or bioflavonoid in place of 2-O-α-D-glucosyl-L-ascorbic acid or α-glucosyl bioflavonoid in the above hair restorer was used.

The results were as shown in Table 2.

**Table 2**

| Effective ingredients | Therapeutic efficacy (Number of volunteers) | | | | Judgement |
|---|---|---|---|---|---|
| | Recovery | Moderate recovery | Not effected | Unfavorable | |
| 2-O-α-D-Glucosyl-L-ascorbic acid | 6 | 10 | 4 | 0 | Present Invention |
| α-Glucosyl rutin | 6 | 12 | 2 | 0 | Present Invention |
| α-Glucosyl hesperidin | 4 | 11 | 5 | 0 | Present Invention |
| α-Glucosyl naringin | 4 | 9 | 7 | 0 | Present Invention |
| L-Ascorbic acid | 0 | 3 | 11 | 6 | Control |
| Rutin | 0 | 4 | 13 | 3 | Control |
| Hesperidin | 0 | 3 | 12 | 5 | Control |
| Naringin | 0 | 5 | 10 | 5 | Control |

As evident from the results in Table 2, the α-glycosyl-L-ascorbic acid and α-glycosyl bioflavonoid in the invention exhibited a wide variety of effects on alopecias, and, especially they exhibited a satisfiable effect against the male alopecia such as senile alopecia and alopecia prematura, as well as alopecia areata. Furthermore, no side effect of the α-glycosyl-L-ascorbic acid and α-glycosyl bioflavonoid was found.

Based on the results of this Experiment, the effect of the effective ingredients such as 2-O-α-D-glucosyl-L-ascorbic acid, α-glucosyl rutin, α-glucosyl hesperidin, and α-glucosyl naringin was evaluated based on the cure rate (%), a percentage of the number of volunteers who answered "recovery" and "moderate recovery" against the total number of each group that received one of the effective ingredients. The cure rates (%) 2-O-α-D-glucosyl-L-ascorbic acid, α-glucosyl rutin, α-glucosyl hesperidin, and α-glucosyl naringin were calculated as 80%, 90%, 75% and 65%, respectively.

The efficacy of the group with either intact L-ascorbic acid or bioflavonoid was low as indicated by the cure rate (%) in the range of about 15-25%, and there were many volunteers who developed inflamation, dandruff and itching of the scalp, or unsatisfactory feeling.

### Experiment 3

An acute toxicity was tested for an α-glycosyl-L-ascorbic acid specimen and an α-glycosyl bioflavonoid specimen, which had been prepared by the methods in Examples of Preparation 1-8, using dd-mice (7-weeks old) by orally administering either of them to the mice. As a result, no mice was died even with a dose of 5g/mouse, and a higher dose test was impossible.

Furthermore, the skin-stimutativeness of the hair restorers in Experiment 1 were respectively tested according to the patch test by administering the hair restorers to 30 healthy volunteers. The results showed that all of the hair restorers tested were negative (positive ratio 0%).

Thus, the effective ingredients in the invention are satisfiably high in safeness.

The preparations of α-glycosyl-L-ascorbic acid and α-glycosyl bioflavonoid in the invention will be described hereinafter.

### Example of Preparation 1

### α-Glycosyl-L-ascorbic acid

Forty parts of dextrin (DE about 6) was dissolved in 50 parts of water while heating, and the mixture was added with 13 parts of L-ascorbic acid under reducing conditions. To the resultant was added 270 units/g dextrin solid of cyclomaltodextrin glucanotransferase (EC 2.4.1.19) commercialized by Hayashibara Biochemical Laboratories Inc., Okayama, Japan, and the resultant mixture was allowed to react at pH 5.6 and 65°C for 40 hours under reducing conditions. HPLC analysis of the reaction mixture revealed that about 65% of L-ascorbic acid had been converted into α-glycosyl-L-ascorbic acids such as α-D-glucosyl-L-ascorbic acid, α-maltosyl-L-ascorbic acid, α-maltotriosyl-L-ascorbic acid, αmaltotetraosyl-L-ascorbic acid, α-maltopentaosyl-L-ascorbic acid, and α-maltohexaosyl-L-ascorbic acid.

Thereafter, the reaction mixture was heated to inactivate the remaining enzyme, followed by filtration, which was then decolored and purified in usual manner with an activated charcoal. The resultant was concentrated into an α-glycosyl-L-ascorbic acid syrup containing α-glucosyl saccharides in the yield of about 90% against the material, on the dry solid basis (d.s.b.).

Since the α-glycosyl-L-ascorbic acid in the product exhibits a satisfiable stability and physiological activity, but not exhibit direct reducing activity, it can be advantageously used in hair restorers.

### Example of Preparation 2

### 2-O-α-D-Glucosyl-L-ascorbic acid

One part of an α-glycosyl-L-ascorbic acid syrup containing α-glucosyl saccharides, prepared by the method in Example of Preparation 1, was dissolved in 4 parts of water. To the mixture was added 100 units/g syrup solid of glucoamylase (EC 3.2.1.3) commercialized by Seikagaku Kogyo, Co., Ltd., Tokyo, Japan, and the resultant mixture was allowed to react at 50°C for 5 hours. HPLC analysis of the reaction mixture revealed that α-glycosyl-L-ascorbic acids had been converted into 2-O-α-D-glucosyl-L-ascorbic acid.

The reaction mixture was heated to inactivate the remaining enzyme, followed by filtration. The filtrate was subjected to gel chromatography using water for elution and "Bio-Gel P-2", a gel commercialized by Bio-Rad Laboratories, New York, U.S.A., followed by the recovery of α-D-glucosyl-L-ascorbic acid rich fractions. The fractions were subjected to HPLC using "Shim-pack ODS", a column commercialized by Shimadzu Seisakusho, Ltd., Tokyo, Japan, and 0.3% acetic acid to effect elution, followed by the recovery of α-D-glucosyl-L-ascorbic acid rich fractions. The resultant fractions were concentrated in vacuo, lyophilized, and prepared into a 2-O-α-D-glucosyl-L-ascorbic acid, a purity, 99.0% or higher, in the yield of about 80% against the material L-ascorbic acid, d.s.b.

Since the product contains a highly-purified 2-O-α-D-glucosyl-L-ascorbic acid and exhibits a satisfiable stability and physiological activity, but not exhibit direct reducing activity, it can be advantageously used in hair restorers.

Furthermore, the product can be advantageously used intact, or, if necessary in combination with an electrolyte or cyanine dye as a hair restorer for iontophoresis.

### Example of Preparation 3

### α-Glycosyl rutin

Three parts of rutin and 15 parts of dextrin (DE 18) were mixed in 97 parts of 80°C water to obtain a high-rutin content suspension which was then added with 20 units/g dextrin solid of cyclomaltodextrin glucanotransferase, derived from a microorganism of the species Bacillus stearothermophilus, and allowed to react at pH 6.0 and 75°C for 64 hours. Paper chromatographic analysis of the reaction mixture revealed that about 85% of intact rutin had been converted into α-glycosyl rutins such as α-glucosyl-rutin, α-maltosyl rutin, α-maltotriosyl rutin, α-maltotetraosyl rutin, and α-malto-pentaosyl rutin.

Thereafter, the reaction mixture was heated to inactivate the remaining enzyme, followed by filtration. The filtrate was concentrated into an α-glycosyl rutin syrup containing amylaceous substances in the yield of about 90% against the material, d.s.b.

Since the product is satisfiably high in safeness, relatively high in water-solubility and physiologically active, it can be advantageously used in hair restorers.

### Example of Preparation 4

### α-Glucosyl rutin

One part of an α-glycosyl rutin syrup containing amylaceous substances, prepared by the method in Example of Preparatior 3, was dissolved in 4 parts of water. To the mixture was added 100 units/g syrup solid of glucoamylase, and the resultant mixture was allowed to react at 50°C for 5 hours. Paper chromatographic analysis of the reaction mixture revealed that α-glycosyl rutin had been converted into α-glucosyl rutin.

The reaction mixture was heated to inactivate the remaining enzyme, followed by filtration. The filtrate was fed to a column of "Diaion HP-10", a macroporous synthetic resin commercialized by Mitsubishi Chemical Industries Ltd., Tokyo, Japan, at SV 2. As a result, α-glucosyl rutin and intact rutin in the filtrate were adsorbed onto the resin, while glucose and salts were passed through the column without causing adsorption. Thereafter, the column was first washed by feeding thereto water, then fed with an aqueous ethanol solution while stepwisely increasing its concentration, followed by the recovery of α-glucosyl rutin fractions which were concentrated in vacuo, and prepared into an α-glucosyl rutin powder in the yield of about 80% against the material rutin, d.s.b.

The α-glucosyl rutin formed one mole of L-rhamnose and 2 moles of D-glucose were formed per one mole of quercetin when hydrolyzed with acid. Furthermore, it was found that α-glucosyl rutin hydrolyzed into rutin and D-glucose, when exposed to an α-glucosidase which had been extracted from a pig liver and partially purified.

Since the product is a satisfiably high in safeness, relatively high in water-solubility and physiologically active, it can be advantageously used in hair restorers.

### Example of Preparation 5

### α-Glycosyl hesperidin

One part of hesperidin was dissolved in 4 parts of 1N sodium hydroxide solution, and the mixture was neutralized by the addition of 0.01N hydrochloric acid solution, and further added with 4 parts of dextrin (DE 10). Immediately after that, 20 units/g dextrin solid of cyclodextrin glucanotransferase, derived from a microorganism of the species Bacillus stearothermophilus, was added to the mixture. The resultant mixture was allowed to react at pH 6.0 and 75°C for 24 hours while stirring. Thin-layer chromatographic analysis of the reaction mixture revealed that about 70% hesperidin had been converted into α-glycosyl hesperidins such as α-glucosyl hesperidin, α-maltosyl hesperidin, α-maltotriosyl hesperidin, α-maltotetraosyl hesperidin, and α-maltopentaosyl hesperidin.

Thereafter, the reaction mixture was heated to inactivate the remaining enzyme, followed by filtration. The filtrate was desalted and purified with ion-exchange resins (H- and OH-form), and the resultant solution was concentrated into an α-glycosyl hesperidin syrup containing α-glucosyl saccharides in the yield of about 90% against the material, d.s.b.

Since the product is a satisfiably high in safeness, relatively high in water-solubility and physiologically active, it can be advantageously used in hair restorers.

### Example of Preparation 6

### α-Glucosyl hesperidin

One part of an α-glycosyl hesperidin syrup containing α-glycosyl saccharides, prepared by the method in Example of Preparation 5, was dissolved in 4 parts of water. To the mixture was added 100 units/g syrup solid of glucoamylase, and the resultant mixture was allowed to react at 50°C for 5 hours. Thin-layer chromatographic analysis of the reaction mixture revealed that the α-glycosyl hesperidin had been converted into α-glucosyl hesperidin.

The reaction mixture was heated to inactivate the remaining enzyme, followed by filtration. The filtrate was fed to a column of "Diaion HP-10", a macroporous synthetic resin at SV 2. Thus, α-glucosyl hesperidin and intact hesperidin in the filtrate adsorbed onto the resin, while the glucose and salts were passed through the column without causing adsorption. Thereafter, the column was first washed by feeding thereto water, then fed with an aqueous ethanol solution while stepwisely increasing its concentration, followed by the recovery of α-glucosyl hesperidin rich fractions which were then concentrated in vacuo, and prepared into an α-glucosyl hesperidin powder in the yield of about 60% against the material hesperidin, d.s.b.

The α-glucosyl hesperidin formed one mole of L-rhamnose and 2 moles of D-glucose weere formed per one mole of hesperidin when hydrolyzed with acid. Furthermore, it was found that the α-glucosyl hesperidin formed hesperidin and D-glucose when exposed to an α-glucosidase which had been extracted from a pig lever and partially purified.

Since the product contains a highly-purified α-glucosyl hesperidin which is satisfiably high in safeness, high in water-solubility and physiologically active, it can be advantageously used in hair restorers.

### Example of Preparation 7

### α-Glycosyl naringin

One part of naringin and 4 parts of dextrin (DE 10) were dissolved in 10 parts of water while heating, and the mixture was cooled to 75°C. Immediately after that, 20 units/g dextrin solid of cyclomaltodextrin glucanotransferase, derived from a microorganism of the species Bacillus stearothermophilus, and the resultant mixture was allowed to react at pH 5.5 and 75°C for 24 hours while stirring. Thin-layer chromatographic analysis of the reaction mixture revealed that about 65% of naringin had been converted into α-glycosyl naringins sucti as α-glucosyl naringin, α-maltosyl naringin, α-maltotriosyl naringin, and α-maltotetraosyl naringin.

Thereafter, the reaction mixture was heated to inactivate the remaining enzyme, followed by filtration. The filtrate was desalted and purified in usual manner with ion-exchange resins (H- and OH-form), and concentrated into an α-glycosyl naringin syrup containing α-glucosyl saccharides in the yield of about 85% against the material, d.s.b.

Since the product is satisfiably high in safeness, relatively high in water-solubility and physiologically active, it can be advantageously used in hair restorers.

### Example of Preparation 8

### α-Glucosyl naringin

One part of an α-glycosyl naringin syrup containing α-glucosyl saccharides, prepared by the method in Example of Preparation 7, was dissolved in 4 parts of water. To the mixture was added 100 units/g syrup solid of glucoamylase and the resultant mixture was allowed to react at 50°C for 5 hours. Thin-layer chromatographic analysis of the reaction mixture revealed that the α-glycosyl naringin had been converted into α-glucosyl naringin.

The reaction mixture was heated to inactivate the remaining enzyme, followed by filtration. The filtrate was fed to a column of "Diaion HP-10", a macroporous synthetic resin at SV 2. As a result, α-glucosyl naringin and intact naringin in the filtrate were absorbed onto the resin, while the glucose and salts were passed through the column without causing adsorption. Thereafter, the column was first washed by feeding thereto water, then fed with an aqueous ethanol solution while stepwisely increasing its concentration to recover α-glucosyl naringin rich fractions which were then concentrated in vacuo, and prepared into an α-glucosyl naringin powder in the yield of about 55% against the material naringin, d.s.b.

The α-glucosyl naringin formed one mole of L-rhamnose and 2 moles of D-glucose were formed per one mole of naringenin when hydrolyzed with acid. Furthermore, it was found that an α-glucosidase, which had been extracted from a pig lever and partially purified, hydrolyzed the α-glucosyl naringin into naringin and D-glucose.

Since the product contains a highly-purified αglucosyl naringin which is satisfiably high in safeness, high in water-solubility and physiologically active, it can be advantageously used in hair restorers.

Examples of the hair restorer according to the present invention will be described hereafter.

### [Examples]

### Example 1

### Hair restorer

Fifty-five parts of ethanol, 2.0 parts of polyoxyethylene (8) oleyl alcohol ether, 43.0 parts of refined water, 2.0 parts of an α-glucosyl rutin prepared by the method in Example of Preparation 4, 1.0 part of vitamin E, and adequate amounts of a flavoring agent, β-thujaplicin (hinokitiol) and coloring agent were mixed in usual manner to obtain a hair restorer in hair tonic form.

Since the product contains α-glucosyl rutin and vitamin E, it can be advantageously used in the promotion of the growth and regeneration of hair for human and animals, as well as in the treatment and prevention of falling out of hair, dandruff, and itching of the scalp or skin.

Furthermore, the product can be also used as a hair tonic because the product exhibits an effect of imparting flavor and refreshment. In addition, the product can be advantageously used as a preventive or traumatherapeutic agent for the scalp because α-glucosyl rutin acts as an absorbent of ultraviolet.

### Example 2

### Hair restorer

A hair restorer was prepared similarly as in Example 1, except that 2.0 parts of α-glucosyl rutin in Example 1 was replaced with 3.0 parts of the α-glucosyl-L-ascorbic acid prepared by the method in Example of Preparation 2. Since the product contains the α-glucosyl-L-ascorbic acid, it can be advantageously used in the promotion of the growth and regeneration of hair for human and animals, as well as in the treatment and prevention of falling out of hair, dandruff, and itching of the scalp or skin.

The product can be favorably used as a hair tonic because the product exhibits an effect of imparting flavor and refreshment. Furthermore, the product can be favorably used as a preventive or traumatherapeutic agent for the scalp because the α-glucosyl-L-ascorbic acid acts as an absorbent of ultraviolet and promotes the collagen synthesis by its absorption to the skin.

### Example 3

### Hair restorer

Twenty parts of polyoxypropylene (40) butyl ether, 3.0 parts of an α-glucosyl hesperidin prepared by the method in Example of Preparation 6, 55.0 parts of ethanol, 23.0 parts of refined water, and adequate amounts of a flavoring agent, coloring agent and antiseptic, were mixed in usual manner to obtain a hair restorer in hair liquid form.

Since the product contains α-glucosyl hesperidin, it can be advantageously used in the promotion of the growth and regeneration of hair for human and animals, as well as in the treatment and prevention of falling out of hair, dandruff, and itching of the scalp or skin.

The product can be favorably used as a hair liquid because the product exerts an effect of imparting flavor and refreshment. Furthermore, the product can be also used as a prevertive or traumatherapeutic agent for the scalp because the α-glucosyl hesperidin acts as an absorbent of ultraviolet and promotes the collagen synthesis by its absorption to the skin.

### Example 4

### Hair restorer

Eighty parts of liquid paraffin, 18.0 parts of olive oil, 2.5 parts of an α-glucosyl naringin prepared by the method in Example of Preparation 8, and 1.0 part of a flavoring agent were mixed in usual manner to obtain a hair restorer in hair oil form.

Since the product contains α-glucosyl naringin, it can be advantageously used in the promotion of the growth and regeneration of hair for human and animals, as well as in the treatment and prevention of falling out of hair, dandruff, and itching of the scalp or skin.

Furthermore, the product can be also used as a hair oil because the product imparts a gloss to hair and exerts an antiphlogistic activity.

### Example 5

### Hair restorer

Three parts of beeswax, 3.0 parts of an α-glycosyl rutin prepared by the method in Example of Preparation 3, 15.0 parts of petrolatum, 42.0 parts of liquid paraffin, 3.0 parts of polyoxyethylene (5) ester stearate, 2.0 parts of polyoxyethylene (6) oleyl alcohol ether, 1.0 part of polyoxyethylene (6) cetyl alcohol ether, 34.0 parts of refined water, and adequate amounts of a flavoring agent and antiseptic, were mixed in usual manner to obtain a hair restorer in hair cream form.

Since the product contains α-glycosyl rutin, it can be advantageously used in the promotion of the growth and regeneration of hair for human and animals, as well as in the treatment and prevention of falling out of hair, dandruff, and itching of the scalp or skin.

Furthermore, the product can be also used as a hair cream because the product imparts a gloss to hair, as well as supplementing nutritions to the skin and exerting an antiphlogistic activity.

### Example 6

### Hair restorer

One and half parts of an α-glycosyl-L-ascorbic acid prepared by the method in Example of Preparation 1, 1.0 part of an α-glycosyl hesperidin prepared by the method in Example of Preparation 5, 0.2 parts of alkyldiaminoethylglycine hydrochloride solution, 20.0 parts of lauryl dimethylaminoacetic acid betaine, 25.0 parts of lauryl methyl tauride, refined water, and adequate amounts of an antiseptic and flavoring agent, were dissolved by heating in usual manner to obtain a hair restorer in shampoo form.

Since the product contains α-glycosyl-L-ascorbic acid and α-glycosyl hesperidin, it can be advantageously used in the promotion of the growth and regeneration of hair for human and animals, as well as in the treatment and prevention of falling out of hair, dandruff, and itching of the scalp or skin.

Furthermore, the product can be favorably used as a shampoo which can effectively wash hair without damaging hair.

### Example 7

### Hair restorer

Two parts of an α-glycosyl-L-ascorbic acid prepared by the method in Example of Preparation 1, 2.0 parts of an α-glycosyl rutin prepared by the method in Example of Preparation 3, 2.0 parts of distearylmethylammonium chloride, 2.0 parts of cetanol, 2.0 parts of silicone resin, 1.0 part of polyoxyethylene oleyl alcohol ether, and an adequate amount of a flavoring agent was dissolved by heating. The resultant was added with a mixture consisting of 3.0 parts of 1,3-butylene glycol, 89.0 parts of refined water, and an adequate amount of a flavoring agent under stirring conditions. Thereafter, the resultant mixture was cooled, and allowed to stand to obtain a hair restorer in rinse form.

Since the product is a hair restorer containing α-glycosyl-L-ascorbic acid and α-glycosyl rutin, it can be advantageously used as a hair restorer for human and animals, as well as in the treatment and prevention of falling out of hair, dandruff, and itching of the scalp or skin.

Furthermore, the product can be also used as a rinse because it can effectively rinse hair.

### [Effect of the Invention]

As described above, since the hair restorer according to the present invention which contains α-glycosyl-L-ascorbic acid and/or α-glycosyl bioflavonoid, the hair restorer when applied to human and animals exhibits a satisfiable effect of the growth and regeneration of hair such as capilli, supercilia, moustaches and beards, without unfavorable side effects.

Furthermore, the present hair restorer exhibits a satisfiable effect in the treatment and prevention of cutting off or falling out of hair, dandruff, and itching of the scalp or skin, as well as in the protection of the scalp from ultraviolet and in the traumatherapeutic treatment of the scalp.

Thus, the present hair restorer exhibits the following actual advantages:
(i) The hair restorer treats and prevents human alopecia;
(ii) The hair restorer improves the quality of hair of pet animals; and
(iii) The hair restorer improves the commercial value of fur-bearing animals whose hair and fur are utilized.

It is concluded that the present hair restorer exhibits the above effects because of the following reason:

The α-glycosyl-L-ascorbic acid and α-glycosyl bioflavonoid, which are incorporated as the effective ingredient in the present hair restorer, have a relatively high-stability and high-affinity to human and animal skin, and satisfiably penetrate into a deeper part of skin tissue of human and animals to exhibit the activity inherent to intact vitamins C and P.

## Claims

1. A hair restorer, which contains as the effective ingredient a member selected from alpha-glycosyl-L-ascorbic acid, alpha-glycosyl bioflavonoid, and mixtures thereof, said alpha-glycosyl bioflavonoid is a member selected from alpha-glycosyl rutin, alpha-glycosyl hesperidin, alpha-glycosyl naringin, and mixtures thereof.

2. A hair restorer according to claim 1, wherein said alpha-glycosyl-L-ascorbic acid is a member selected from 2-0-alpha-D-glucosyl-L-ascorbic acid, 2-0-alpha-maltosyl-L-ascorbic acid, 2-0-alpha-maltotriosyl-L-ascorbic acid, 2-0-alpha-maltotetraosyl-L-ascorbic acid, 2-0-alpha-maltopentaosyl-L-ascorbic acid, 2-0-alpha-maltohexaosyl-L-ascorbic acid, 2-0-alpha-maltoheptaosyl-L-ascorbic acid, and mixtures thereof.

3. A hair restorer according to claim 1 or 2, wherein said alpha-glycosyl rutin is a member selected from alpha-glucosyl rutin, alpha-maltosyl rutin, alpha-maltotriosyl rutin, alpha-maltotetraosyl rutin, alpha-maltopentaosyl rutin, and mixtures thereof.

4. A hair restorer according to claim 1, 2 or 3, wherein said alpha-glycosyl hesperidin is a member selected from alpha-glucosyl hesperidin, alpha-maltosyl hesperidin, alpha-maltotriosyl hesperidin, alpha-maltotetraosyl hesperidin, alpha-maltopentaosyl hesperidin, and mixtures thereof.

5. A hair restorer according to any one of claims 1 to 4, wherein said alpha-glycosyl naringin is a member selected from alpha-glucosyl naringin, alpha-maltosyl naringin, alpha-maltotriosyl naringin, alpha-maltotetraosyl naringin, and mixtures thereof.

6. A hair restorer according to any one of the preceding claims, which contains 0.001-10% by weight of said alpha-glycosyl-L-ascorbic acid and/or alpha-glycosyl bioflavonoid in total.

7. A hair restorer according to any one of the preceding claims, which additionally contains 0.001-0.01% by weight of a cyanine dye.

8. The hair restorer according to claim 7, wherein said cyanine dye is 6-[2-[(5-bromo-2-pyridyl)amino]vinyl]-1-ethyl-2-picolinium iodide or 2-(2-anilinovinyl)-3,4-dimethyloxazolium iodide.

9. A hair restorer according to any one of the preceding claims, which is in the form of liquid, jelly, emulsion, aerosol or ointment.

10. A hair restorer according to any one of the preceding claims, which additionally contains an effective amount of humectant.

11. A hair restorer according to claim 10, wherein said humectant is propyleneglycol, 1,3-butyleneglycol, glycerine, sorbitol, 2-octyldodecyl myristate, polyoxyethylene polyoxypropylene glycol, or lauric acid diethanolamine.

12. A cosmetic method for improving the growth and regeneration of hair, said method comprising the step of administering an effective amount of a hair restorer according to any one of the preceding claims.

13. A cosmetic method acccording to claim 12, wherein said hair restorer is administered to an animal in a dose of 0.0001-10g/day/adult.

## Patentansprüche

1. Haarwuchsmittel, das als aktiven Bestandteil ein Mitglied, das aus alpha-Glycosyl-L-ascorbinsäure, alpha-Glycosylbioflavonoid und Mischungen davon gewählt ist, enthält, wobei das alpha-Glycosylbioflavonoid ein Mitglied, das aus alpha-Glycosylrutin, alpha-Glycosylhesperidin, alpha-Glycosylnaringin und Mischungen davon gewählt ist, ist.

2. Haarwuchsmittel nach Anspruch 1, worin die alpha-Glycosyl-L-ascorbinsäure ein Mitglied ist, das aus 2-0-alpha-D-Glycosyl-L-ascorbinsäure, 2-0-alpha-Maltosyl-L-ascorbinsäure, 2-0-alpha-Maltotriosyl-L-ascorbinsäure, 2-0-alpha-Maltotetraosyl-L-ascorbinsäure, 2-0-alpha-Maltopentaosyl-L-ascorbinsäure, 2-0-alpha-Maltohexaosyl-L-ascorbinsäure, 2-0-alpha-Maltoheptaosyl-L-ascorbinsäure und Mischungen davon gewählt ist.

3. Haarwuchsmittel nach Anspruch 1 oder 2, worin das alpha-Glucosylrutin ein Mitglied ist, das aus alpha-Glucosylrutin, alpha-Maltosylrutin, alpha-Maltotriosylrutin, alpha-Maltotetraosylrutin, alpha-Maltopentaosylrutin und Mischungen davon gewählt ist.

4. Haarwuchsmittel nach Anspruch 1, 2 oder 3, worin das alpha-Glycosylhesperidin ein Mitglied ist, das aus alpha-Glucosylhesperidin, alpha-Maltosylhesperidin, alpha-Maltotriosylhesperidin, alpha-Maltotetraosylhesperidin, alpha-Maltopentaosylhesperidin und Mischungen davon gewählt ist.

5. Haarwuchsmittel nach einem der Ansprüche 1 bis 4, worin das alpha-Glycosylnaringin ein Mitglied ist, das aus alpha-Glucosylnaringin, alpha-Maltosylnaringin, alpha-Maltotriosylnaringin, alpha-Maltotetraosylnaringin und Mischungen davon gewählt ist.

6. Haarwuchsmittel nach einem der vorangegangenen Ansprüche, welches insgesamt 0,001 - 10 Gew.-% der alpha-Glycosyl-L-ascorbinsäure und/oder des alpha-Glycosylflavonoids enthält.

7. Haarwuchsmittel nach einem der vorangegangenen Ansprüche, das weiterhin 0,001 - 0,01 Gew.-% eines Cyaninfarbstoffs enthält.

8. Haarwuchsmittel nach Anspruch 7, worin der Cyaninfarbstoff 6-[2-[(5-Brom-2-pyridyl)amino]vinyl]-1-ethyl-2-picoliniumjodid oder 2-(2-Anilinovinyl)-3,4-dimethyloxazoliumjodid ist.

9. Haarwuchsmittel nach einem der vorangegangen Ansprüche, welches im Form einer Flüssigkeit, eines Gelees, einer Emulsion, eines Aerosols oder einer Salbe vorliegt.

10. Haarwuchsmittel nach einem der vorangegangenen Ansprüche, welches weiterhin eine wirksame Menge eines Feuchthaltemittels enthält.

11. Haarwuchsmittel nach Anspruch 10, worin das Feuchthaltemittel Propylenglykol, 1,3-Butylenglykol, Glyzerin, Sorbit, 2-Octyldodecylmyristat, Polyoxyethylenpolyoxypropylenglykol oder Laurinsäurediethanolamin ist.

12. Kosmetisches Verfahren zur Verbesserung des Wachstums und der Regeneration von Haaren, wobei das Verfahren die Stufe umfaßt, bei der eine wirksame Menge eines Haarwuchsmittels nach einem der vorangegangenen Ansprüche verabreicht wird.

13. Kosmetisches Verfahren nach Anspruch 12, worin das Haarwuchsmittel einem Tier in einer Dosis von 0,0001 - 10 g/Tag/Erwachsener verabreicht wird.

## Revendications

1. Agent de régénération capillaire contenant, comme ingrédient actif, un élément choisi parmi un acide alpha-glycosyl-L-ascorbique, un alpha-glycosyl-bioflavonoïde, et des mélanges de ceux-ci, ledit alpha-glycosyl-bioflavonoïde étant un élément choisi par l'alpha-glycosyl-rutine, l'alpha-glycosyl-hespéridine, l'alpha-glycosyl-naringine, et des mélanges de celles-ci.

2. Agent de régénération capillaire selon la revendication 1, dans lequel ledit acide alpha-glycosyl-L-ascorbique est un élément choisi parmi l'acide 2-0-alpha-D-glucosyl-L-ascorbique, l'acide 2-0-alpha-maltosyl-L-ascorbique, l'acide 2-0-alpha-maltotriosyl-L-ascorbique, l'acide 2-0-alpha-maltotétraosyl-L-ascorbique, l'acide 2-0-alpha-maltopentaosyl-L-ascorbique, l'acide 2-0-alpha-maltohexaosyl-L-ascorbique, l'acide 2-0-alpha-maltoheptaosyl-L-ascorbique, et des mélanges de ceux-ci.

3. Agent de régénération capillaire selon la revendication 1 ou 2, dans lequel ladite alpha-glycosyl-rutine est un élément choisi parmi l'alpha-glucosyl-rutine, l'alpha-maltosyl-rutine, l'alpha-maltotriosyl-rutine, l'alpha-maltotétraosyl-rutine, l'alpha-maltopentaosyl-rutine, et des mélanges de celles-ci.

4. Agent de régénération capillaire selon la revendication 1, 2 ou 3, dans lequel ladite alpha-glycosyl-hespéridine est un élément choisi parmi l'alpha-glucosyl-hespéridine, l'alpha-maltosyl-hespéridine, l'alpha-maltotriosyl-hespéridine, l'alpha-maltotétraosyl-hespéridine, l'alpha-maltopentaosyl-hespéridine, et des mélanges de celles-ci.

5. Agent de régénération capillaire selon l'une quelconque des revendications 1 à 4, dans lequel ladite alpha-glycosyl-naringine est un élément choisi parmi l'alpha-glucosyl-naringine, l'alpha-maltosyl-naringine, l'alpha-maltotriosyl-naringine, l'alpha-maltotétraosyl-naringine, et des mélanges de celles-ci.

6. Agent de régénération capillaire selon l'une quelconque des revendications précédentes, contenant en tout de 0,001 à 10 % en poids dudit acide alpha-glycosyl-L-ascorbique et/ou dudit alpha-glycosyl-bioflavonoïde.

7. Agent de régénération capillaire selon l'une quelconque des revendications précédentes, contenant, en outre, de 0,001 à 0,01 % en poids d'un colorant cyanine.

8. Agent de régénération capillaire selon la revendication 7, dans lequel ledit colorant cyanine est l'iodure de 6-[2-[(5-bromo-2-pyridyl)amino]vinyl]-1-éthyl-2-picolinium ou l'iodure de 2-(2-anilinovinyl)-3,4-diméthyloxazolium.

9. Agent de régénération capillaire selon l'une quelconque des revendications précédentes, sous la forme d'un liquide, d'un gel, d'une émulsion, d'un aérosol ou d'un onguent.

10. Agent de régénération capillaire selon l'une quelconque des revendications précédentes, contenant, en outre, une quantité efficace d'agent hydratant.

11. Agent de régénération capillaire selon la revendication 10, dans lequel ledit agent hydratant est le propylèneglycol, le 1,3-butylèneglycol, le glycérol, le sorbitol, le myristate de 2-octyldodécyle, un polyoxyéthylène-polyoxypropylène-glycol ou la diéthanolamine de l'acide laurique.

12. Procédé cosmétique pour améliorer la croissance et la régénération des cheveux, ledit procédé comprenant l'étape consistant à administrer une quantité efficace d'un agent de régénération capillaire selon l'une quelconque des revendications précédentes.

13. Procédé cosmétique selon la revendication 12, selon lequel ledit agent de régénération capillaire est administré à un animal à une dose de 0,0001 à 10 g/jour/adulte.
